# EUROPEAN PATENT SPECIFICATION

(11) **EP 1 687 036 B1**
(45) Date of publication and mention of the grant of the patent: **20.08.2008**
(21) Application number: 04812130.5
(22) Date of filing: 24.11.2004
(51) Int. Cl.: A61L 12/08

(54) **STABILITY ENHANCEMENT OF SOLUTIONS CONTAINING ANTIMICROBIAL AGENTS**
STABILITÄTSVERBESSERUNG VON LÖSUNGEN MIT ANTIMIKROBIELLEN MITTELN
AMELIORATION DE STABILITE DE SOLUTIONS CONTENANT DES AGENTS ANTIMICROBIENS

(30) Priority: 01.12.2003 US 725233
(43) Date of publication of application: 09.08.2006
(73) Proprietor: BAUSCH & LOMB INCORPORATED, Rochester, NY 14604 (US)
(72) Inventor: QUENVILLE, Irene, Oswego, NY 13126 (US); XIA, Erning, Penfield, NY 14526 (US); MAIER, Stephen, Brockport, NY 14420 (US); LEVER, O. William, Jr., Pittsford, NY 14534 (US); HEILER, David, J., Avon, NY 14414 (US); DOBIE, Alyce, K., Williamson, NY 14589 (US)
(74) Representative: Maiwald Patentanwalts GmbH
(86) International application number: PCT/US2004/039547
(87) International publication number: WO 2005/053757

(56) References cited:
- US-B1- 6 228 323

## Description

### Field of the Invention:

The present invention relates to stability enhancement of compositions useful for cleaning and disinfecting contact lenses. More specifically, the present invention relates to lens care solutions produced from compositions containing poly(hexamethylene biguanide) for cleaning and disinfecting contact lenses and the use of a container formed of poly(ethylene terephthalate) to enhance solution stability and increase solution shelf-life.

### Background of the Invention:

Conventionally, contact lenses have been classified into water-nonabsorptive contact lenses and water-absorptive contact lenses, and classified into hard contact lenses and soft contact lenses. Both hard and soft contact lenses may develop deposits or stains of proteins and/or lipids while the lens is worn in the eye. Such stains may cause a deterioration in the comfort of a lens during wear or cause eye problems such as blurred eyesight or congestion of the cornea. Accordingly, it is essential to apply a cleaning treatment to a contact lens in order to safely and comfortably use contact lenses every day.

To effectively clean contact lenses, solutions formulated for cleaning contact lenses having cleaning or removal effect over one or more stains are typically used. Solutions formulated for cleaning contact lenses may include therein a surfactant useful as a cleaning component. Contact lens cleaning solutions incorporating nonionic surfactants such as a polyoxyalkylene block copolymer such as a polyoxyethylene-polyoxypropylene block copolymer or a derivative thereof are known.

Contact lens care solutions also typically include antimicrobial agents for the purpose of disinfecting contact lenses or for the purpose of preserving the solution. Antimicrobial agents are present in such solutions at levels that ensure biocidal efficacy throughout the product or solution shelf-life.

In packaging contact lens care solutions, high density polyethylene (HDPE) bottles are standard. HOPE bottle resins contain numerous additives, such as antioxidants, plasticizers, flame retardants, and the like. HDPE bottle resin additives have the ability to migrate or "bloom" to the surfaces of the bottle and potentially interact with lens care solution ingredients. This "blooming" phenomenon of HOPE resin additives is typically exacerbated by the presence of surfactants, such as those found useful as cleaning components in lens care solutions.

Accordingly, it would be desirable to have a material for contact lens care solution packaging that does not contain numerous additives that tend to migrate or bloom to the surfaces of said packaging.

### Summary of the Invention:

The present invention provides packaging in the form of clear bottles produced from poly(ethylene terephalate) (PET) resin useful in packaging lens care solutions, which include surfactants and poly(hexamethylene biguanide). Unexpectedly, significant improvements in chemical stability and disinfection efficacy were observed In such lens care solutions packaged in PET bottles.

### Brief Description of the Drawings:

FIGURE 1 is a graph depicting antimicrobial agent stability profile of Test Solution 1 in PET vs. HDPE packaging;
FIGURE 2 is a graph depicting antimicrobial agent stability profile of Test Solution 2 in PET vs. HDPE packaging;
FIGURE 3 is a bar chart illustrating biocidal efficacy of Test Solution 1 against *Fusarium solani* in PET vs. HDPE packaging; and
FIGURE 4 is a bar chart illustrating biocidal efficacy of Test Solution 2 against *Candida albicans* in PET vs. HDPE packaging.

### Detailed Description of the Invention:

The present invention provides lens care solution packaging in the form of clear bottles produced from poly(ethylene terephalate) (PET) resin. Contact lens care solutions comprising one or more cleaning surfactants and poly(hexamethylene biguanide) unexpectantly have been found to have enhanced chemical stability and enhanced biocidal efficacy when packaged in containers such as but not limited to bottles formed from. PET resin.

Compositions found to have enhanced properties when packaged in PET containers are aqueous solutions. Such compositions may include one or more nonionic polyether surfactants. Suitable nonionic polyether surfactants for use in compositions of the present invention include for example but are not limited to Pluronic P123^{™} (BASF, Mount Olive, New Jersey) having a hydrophilic/lipophilic balance (HLB) of 8, Pluronic L42^{™} (BASF) having a HLB of 8, Pluronic L62^{™} (BASF) having a HLB of 7, Pluronic L72^{™} (BASF) having a HLB of 7, Pluronic L92^{™} (BASF) having a HLB of 6, Pluronic P103^{™} (BASF) having a HLB of 9, Pluronic R 12R3^{™} (BASF) having a HLB of 7, Pluronic R 17R1^{™} (BASF) having a HLB of 3, Pluronic R 17R2^{™} (BASF) having a HLB of 6, Pluronic R 31R1^{™} (BASF) having a HLB of 1, Pluronic R 31R2^{™} (BASF) having a HLB of 2, Pluronic R 31R4^{™} (BASF) having a HLB of 7, Tetronic 701^{™} (BASF) having a HLB of 3, Tetronic 702^{™} (BASF) having a HLB of 7, Tetronic 901^{™} (BASF) having a HLB of 3, Tetronic 1101^{™} (BASF) having a HLB of 2, Tetronic 1102^{™} (BASF) having a HLB of 6, Tetronic 1301^{™} (BASF) having a HLB of 2, Tetronic 1302^{™} (BASF) having a HLB of 6, Tetronic 1501^{™} (BASF) having a HLB of 1, Tetronic 1502^{™} (BASF) having a HLB of 5, Tetronic R 50R1^{™} (BASF) having a HLB of 3, Tetronic R 50R4^{™} (BASF) having a HLB of 9, Tetronic R 70R1^{™} (BASF) having a HLB of 3, Tetronic R 70R2^{™} (BASF) having a HLB of 5, Tetronic R 70R4^{™} (BASF) having a HLB of 8, Tetronic R 90R1^{™} (BASF) having a HLB of 2, Tetronic R 90R4^{™} (BASF) having a HLB of 7, Tetronic R 110R1^{™} (BASF) having a HLB of 2, Tetronic R 110R2^{™} (BASF) having a HLB of 4, Tetronic R 110R7^{™} (BASF) having a HLB of 10, Tetronic R 130R1^{™} (BASF) having a HLB of 1, Tetronic R 130R2^{™} (BASF) having a HLB of 3, Tetronic R 150R1^{™} (BASF) having a HLB of 1, Tetronic R 150R4^{™} (BASF) having a HLB of 5 and Tetronic R 150R8^{™} (BASF) having a HLB of 11. Such nonionic polyether surfactants are preferably employed in compositions of the present invention in amounts ranging from 0.1 to 6.0 weight percent, more preferably from 0.2 to 5.0 weight percent to achieve cleaning efficacy.

Compositions found to have enhanced properties when packaged in PET containers include quaternary ammonium salts that do not include significant hydrophobic portions, e.g., alkyl chains comprising more than six carbon atoms. Suitable quaternary ammonium salts for use in the present invention include poly(hexamethylene biguanide) (PHMB) available from ICI Americas. Inc., Wilmington, Delaware under the trade name Cosmocil CQ,.

Poly(hexamethylene biguanide) is present in the subject compositions in an amount effective for disinfecting a contact lens, as found in conventional lens soaking and disinfecting solutions. Preferably, poly(hexamethylene biguanide) will be used in a disinfecting amount or an amount from 0.0001 to 0.5 weight percent by volume. A disinfecting amount of poly(hexamethylene biguanide) is an amount that will at least partially reduce the microorganism population in the formulations employed. Preferably, a disinfecting amount is that which will reduce the microbial burden by two log orders in four hours and more preferably by one log order In one hour. Most preferably, a disinfecting amount is an amount that will eliminate the microbial burden on a contact lens when used in the regimen for the recommended soaking time (FDA Chemical Disinfection Efficacy Test - July, 1985 Contact Lens Solution Draft Guidelines). Typically, poly(hexamethylene biguanide) is present in concentrations ranging from 0.00001 to 0.5 weight percent based on volume (w/v), and more preferably, from 0.00003 to 0.05 weight percent.

Compositions having enhanced properties when packaged in PET containers may also contain various other components including for example, but not limited to one or more chelating and/or sequestering agents, one or more osmolarity adjusting agents, one or more surfactants, one or more buffering agents and/or one or more wetting agents.

Chelating agents, also referred to as sequestering agents, are frequently employed in conjunction with an antimicrobial agent. These agents bind heavy metal ions that might otherwise react with the lens and/or protein deposits and collect on the lens. Chelating agents are well known in the art, and examples of preferred chelating agents include ethylenedlamlnetetraacetic acid (EDTA) and its salts, especlally disodium EDTA. Such agents are normally employed in amounts from 0.01 to 2.0 weight percent, more preferably from 0.01 to 0.3 weight percent. Other suitable sequestering agents include for example gluconic acid, citric acid, tartaric acid and their salts, e.g., sodium salts.

Compositions having enhanced properties when packaged in PET containers may be designed for a variety of osmolarities, but it is preferred that the compositions are iso-osmal with respect to eye fluids. Specifically, it is preferred that the compositions have an osmotic value of less than 350 mOsm/kg, more preferably from 175 to 330 mOsm/kg, and most preferably from 260 to 310 mOsm/Kg. One or more osmolarity adjusting agents may be employed in the composition to obtain the desired final osmolarity. Examples of suitable osmolarity adjusting agents include, but are not limited to sodium and potassium chloride, monosaccharides such as dextrose, calcium and magnesium chloride, and low molecular weight polyols such as glycerin and propylene glycol. Typically, these agents are used individually in amounts ranging from 0.01 to 5 weight percent and preferably, from 0.1 to 2 weight percent

Compositions having enhanced properties when packaged in PET containers preferably have an ophthalmically compatible pH, which generally will range between 6 to 8, and more preferably between 6.5 to 7.8, and most preferably 7 to 7.5. One or more conventional buffers may be employed to obtain the desired pH value. Suitable buffers include for example but are not limited to borate buffers based on boric acid and/or sodium borate, phosphate buffers based on Na₂HPO₄, NaH₂PO₄ and/or KH₂PO₄, citrate buffer based on potassium citrate and/or citric acid, sodium bicarbonate and combinations thereof. Generally, buffers will be used in amounts ranging from 0.05 to 2.5 weight percent, and preferably, from 0.1 to 1.5 weight percent.

Such compositions may likewise include a wetting agent to facilitate the composition wetting the surface of a contact lens. Within the art, the term "humectant" is also commonly used to describe these materials. A first class of wetting agents is polymer wetting agents. Examples include for example but are not limited to polyvinyl alcohol (PVA), polyvinyl pyrrolidone (PVP), cellulose derivatives and polyethylene glycol. Cellulose derivatives and PVA may be used to also increase viscosity of the composition, and offer this advantage if desired. Specific cellulose derivatives include for example but are not limited to hydroxypropyl methyl cellulose, carboxymethyl cellulose, methyl cellulose, hydroxyethyl cellulose, and cationic cellulose derivatives. As disclosed in U.S. Patent No. 6,274,133, cationic cellulosic polymers help prevent the accumulation of lipids and proteins on a hydrophilic lens surface. Such polymers include commercially available water soluble polymers available under the CTFA (Cosmetic, Toiletry, and Fragrance Association) designation Polyquatemium-10, including the cationic cellulosic polymers available under the trade name CARE® Polymer from Amerchol Corp., Edison, New Jersey. Generally, these cationic cellulose polymers contain quatemized N,N-dimethyl amino groups along the cellulosic polymer chain.

Another suitable class of wetting agents is non-polymeric wetting agents. Examples include glycerin, propylene glycol, and other non-polymeric diols and glycols.

The specific quantities of wetting agents used in the present invention will vary depending upon the application. However, the wetting agents will typically be included in an amount from 0.01 to 5 weight percent, preferably from 0.1 to 2 weight percent.

It will be understood that some composition constituents possess more than one functional attribute. For example, cellulose derivatives are suitable polymeric wetting agents, but are also referred to as "viscosity increasing agents" to increase viscosity of the composition if desired. Glycerin is a suitable non-polymeric wetting agent but is also may contribute to adjusting tonicity.

Compositions found to have enhanced properties when packaged in PET containers may also include at least one ophthalmically acceptable surfactant, which may be either cationic, anionic, nonionic or amphoteric. Preferred surfactants are amphoteric or nonionic surfactants. The surfactant should be soluble in the aqueous solution and non-irritating to eye tissues. The surfactant serves mainly to facilitate removal of non-proteinaceous matter on the contact lens.

Many nonionic surfactants comprise one or more chains or polymeric components having oxyalkylene (-O-R-) repeat units wherein R has 2 to 6 carbon atoms. Representative non-ionic surfactants comprise block polymers of two or more different kinds of oxyalkylene repeat units, which ratio of different repeat units determines the HLB of the surfactant. Typical HLB values for surfactants found to be suitable are in the range of 18 or above. Examples of such poloxamers are polyoxyethylene, polyoxypropylene block copolymers available under the trade name Pluronlc (BASF). Poloxamines are ethylene diamine adducts of such polyoxyethylene, polyoxypropylene block copolymers available under the trade name Tetronic (BASF), including poloxamine 1107 (Tetronic 1107 having a molecular weight from about 7,500 to about 27,000 wherein at least 40 weight percent of said adduct is poly(oxyethylene) having a HLB of 24. Other suitable non-ionic surfactants include for example but are not limited to polyethylene glycol esters of fatty acids, e.g. coconut, polysorbate, polyoxyethylene or polyoxypropylene ethers of higher alkanes (C₁₂-C₁₈), polysorbate 20 available under the trade name Tween® 20 (ICI Americas, Inc.), polyoxyethylene (23) lauryl ether available under the trade name Brij® 35 (ICI Americas, Inc.), polyoxyethyene (40) stearate available under the trade name Myrj® 52 (ICI Americas, Inc.) and polyoxyethylene (25) propylene glycol stearate available under the trade name Atlas® G 2612 (ICI Americas, Inc.).

Another useful class of surfactants are the hydroxyalkylphosphonates, such as those disclosed in U.S. Patent No. 5,858,937 (Richards et al.), and available under the trade name Dequest® (Montsanto Co., St. Louis, Missouri).

Amphoteric surfactants suitable for use in a composition according to the present invention Include materials of the type are offered commercially under the trade name Miranol^{™} (Noveon, Inc., Cleveland, Ohio). Another useful class of amphoteric surfactants is exemplified by cocoamidopropyl betaine, commercially available from various sources.

Various other ionic as well as amphoteric and anionic surfactants suitable for such compositions can be readily ascertained, in view of the foregoing description, from McCutcheon's Detergents and Emulsiflers, North American Edition, McCutcheon Division, MC Publishing Co., Glen Rock, NJ 07452 and the CTFA Intemational Cosmetic Ingredient Handbook, Published by The Cosmetic, Toiletry, and Fragrance Association, Washington, D.C.

Preferably, the surfactants, when present, are employed in a total amount from 0.01 to 15 weight percent, preferably 0.1 to 9.0 weight percent, and most preferably 0.1 to 7.0 weight percent.

As an illustration of the present invention, several examples are provided below. These examples serve only to further illustrate aspects of the invention and should not be construed as limiting the invention.

### EXAMPLE 1 - Preparation of Test Solutions:

Sample solutions for testing were prepared in accordance with the formulations set forth below in Table 1.

**TABLE 1**

| **Ingredients %W/W** | **Test Solution 1** |
|---|---|
| Pluronic F127 | 2.0000 |
| Tetronic 1107 | 1.0000 |
| Boric Acid | 0.8500 |
| Monosodium Phosphate | 0.1500 |
| Disodium Phosphate | 0.3100 |
| Hydroxyalkylphosphonate | 0.1000 |
| PHMB (ppm) | 1.2 |
| (ppm) Polymer JR 30M | 0.0200 |
| Sodium Chloride | 0.1917 |
| Purified Water | Q.S. to 100 gm |

| | |
|---|---|
| Pluronic F127 (BASF) Tetronic 1107 (BASF) Polymer JR 30M (Amerchol Corp.) | |

### EXAMPLE 2 - Test Solution 1 Stability Profile:

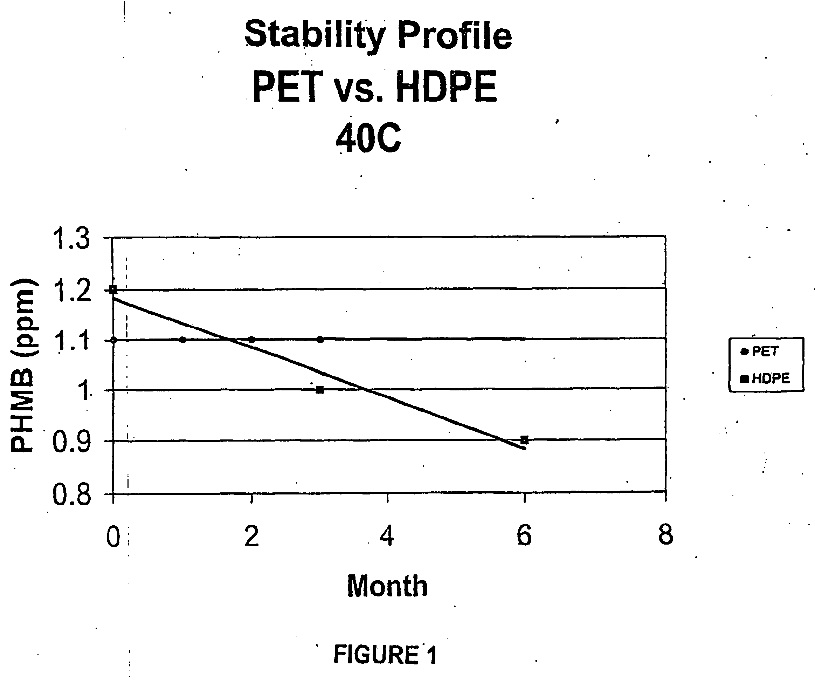

120 ml of Test Solution 1 was filled into each of three 4-ounce PET 7352 containers and three 4-ounce Marlex 5502BN HDPE containers and then stored at 40 °C. Data was collected upon initiation and each month for six months. Collected data is set forth below in Table 2 and illustrated in Figure 1.

**TABLE 2**

| **Test Solution 1** | | |
|---|---|---|
| | **PHMB In ppm** | |
| **Month** | **PET Container** | **HDPE Container** |
| 0 | 1.2 | 1.2 |
| | 1.1 | 1.2 |
| | 1.0 | 1.3 |
| 1 | ND | ND |
| | 1.1 | ND |
| | ND | ND |
| 2 | 1.2 | ND |
| | 1.1 | ND |
| | 1.1 | ND |
| 3 | 1.2 | 1.0 |
| | 1.1 | 1.0 |
| | 1.1 | 1.1 |
| 4 | 1.1 | ND |
| | 1.2 | ND |
| | 1.2 | ND |
| 5 | ND | ND |
| | ND | ND |
| | ND | ND |
| 6 | ND | 1.0 |
| | ND | 0.9 |
| | ND | 0.8 |

### EXAMPLE 3 - Test Solution 1 ISO Stand-Alone Biocidal Efficacy Profile:

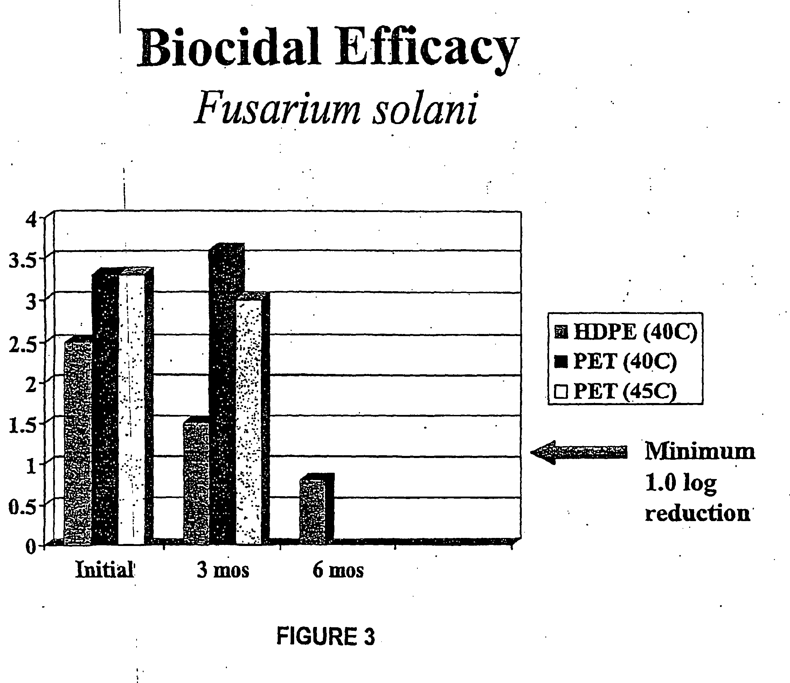

An ISO Stand-Alone Biocidal Efficacy study using 10 percent organic soil was conducted using Test Solution 1, whereby Test Solution 1 was tested against *Staphococcus* aureus ATCC 6538 (bacteria) and *Fusarium solani* ATCC 36031 (mold). The results of the Stand-Alone Biocidal Efficacy study are set forth below in Table 4 and illustrated in Figure 3.

**TABLE 3**

| **Test Solution 1 ISO Stand-Alone Biocidal Efficacy Using 10 Percent Organic Soll and Accelerated Conditions (40 °C)** | | | | | | |
|---|---|---|---|---|---|---|
| **Container** | **initial** | | **3 months** | | **6 months** | |
| | **Sa** | **Fs** | **Sa** | **Fs** | **Sa** | **Fs** |
| HDPE | 4.8 | 2.4 | 4.3 | 2.0 | 3.1 | 0.5 |
| | 4.8 | 2.2 | 4.7 | 1.2 | 2.9 | 1.1 |
| | 4.7 | 3.0 | >4.6 | 1.3 | 1.4 | 0.3 |
| Average | 4.8 | 2.5 | 4.5 | 1.5 | 2.5 | 0.7 |
| PET | >4.9 | 3.8 | >4.6 | 3.2 | >4.8 | 1.7 |
| | 4.4 | 3.2 | 4.9 | 3.8 | ND | ND |
| | 4.0 | 3.0 | >4.9 | 3.1 | >4.7 | 2.8 |
| Average | 4.4 | 3.3 | 4.8 | 3.4 | 4.8 | 2.3 |

| | | | | | | |
|---|---|---|---|---|---|---|
| ND = No Data Sa = *Staphococcus aureus* Fs = *Fusarium solani* | | | | | | |

Based on the findings of the above studies, the present invention comprises a method of enhancing antimicrobial efficacy of a lens care solution comprising packaging.said solution in a container formed of PET resin.

Another method of the present invention comprises enhancing lens care solution stability and hence product shelf-life by packaging said solution in a container formed of PET resin.

Compositions useful as lens care solutions packaged in containers formed from PET resin as described in the present specification, may be packaged, sterilized and used in accordance with methods customary in the field of contact lens care.

Although various preferred embodiments have been illustrated, many other modifications and variations of the present invention are possible to the skilled practitioner. It is therefore understood that, within the scope of the claims, the present invention can be practiced other than as herein specifically described.

## Claims

1. The use of a container formed of poly(ethylene terephthalate) for enhancing stability of a lens care solution containing one or more surfactants and poly(hexamethylene biguanide).

2. The use of claim 1, wherein the solution further comprises at least one member selected from the group consisting of a buffering agent, a chelating agent and an osmolarity adjusting agent.

3. The use of claim 1, wherein the solution comprises 0.0001 to 0.5 weight percent of poly(hexamethylene biguanide).

4. The use of claim 1, wherein the solution further comprises a chelating agent and a buffering agent selected from the group consisting of borate buffers, phosphate buffers and citrate buffers.

5. The use of claim 1, wherein the surfactant has a HLB value of 18 or greater.

6. The use of claim 1, wherein the solution comprises at least one member selected from the group consisting of poloxamer and poloxamine surfactants having a HLB value of 18 or greater.

7. The use of claim 1, wherein the solution comprises 0.1 wt.-% to 2 wt.-% hydroxypropylmethyl cellulose.

8. The use of claim 1, wherein the solution comprises 0.1 wt.-% to 2 wt.-% propylene glycol.

## Patentansprüche

1. Die Verwendung eines Behälters, der aus Polyethylenterephthalat gebildet ist, zur Verbesserung der Stabilität einer Linsenpflegelösung, die ein oder mehrere Tenside und Polyhexamethylenbiguanid enthält.

2. Die Verwendung gemäß Anspruch 1, wobei die Lösung ferner mindestens ein Mitglied ausgewählt aus der Gruppe bestehend aus einer Puffersubstanz, einem Chelatbildner und einer osmolaritätsregulierenden Substanz umfasst.

3. Die Verwendung gemäß Anspruch 1, wobei die Lösung 0,0001 bis 0,5 Gew.-% Polyhexamethylenbiguanid umfasst.

4. Die Verwendung gemäß Anspruch 1, wobei die Lösung ferner einen Chelatbildner und eine Puffersubstanz ausgewählt aus der Gruppe bestehend aus Boratpuffern, Phosphatpuffern und Citratpuffern umfasst.

5. Die Verwendung gemäß Anspruch 1, wobei das Tensid einen HLB-Wert von 18 oder mehr aufweist.

6. Die Verwendung gemäß Anspruch 1, wobei die Lösung mindestens ein Mitglied ausgewählt aus der Gruppe bestehend aus Poloxamer- und Poloxamin-Tensiden mit einem HLB-Wert von 18 oder größer umfasst.

7. Die Verwendung gemäß Anspruch 1, wobei die Lösung 0,1 Gew.-% bis 2 Gew.-% Hydroxypropylmethylcellulose umfasst.

8. Die Verwendung gemäß Anspruch 1, wobei die Lösung 0,1 Gew.-% bis 2 Gew.-% Propylenglykol umfasst.

## Revendications

1. Utilisation d'un conteneur constitué de poly(téréphtalate d'éthylène) pour améliorer la stabilité de solutions de conservation de lentilles contenant un ou plusieurs tensio-actifs et du poly(hexaméthylène biguanide).

2. Utilisation selon la revendication 1, dans laquelle la solution comprend en outre au moins un élément choisi dans le groupe consistant en un tampon, un agent chélatant et un agent d'ajustement de l'osmolarité.

3. Utilisation selon la revendication 1, dans laquelle la solution comprend 0,0001 à 0,5 % en poids de poly(hexaméthylènebiguanide).

4. Utilisation selon la revendication 1, dans laquelle la solution comprend en outre un agent chélatant et un tampon choisis dans le groupe formé par les tampons borate, les tampons phosphate et les tampons citrate.

5. Utilisation selon la revendication 1, dans laquelle le tensio-actif a une valeur HLB de 18 ou plus.

6. Utilisation selon la revendication 1, dans laquelle la solution comprend au moins un élément choisi dans le groupe formé par les tensioactifs poloxamères et poloxamines ayant une valeur HLB de 18 ou plus.

7. Utilisation selon la revendication 1, dans lequel la solution comprend 0,1 % en poids à 2 % en poids d'hydroxypropylméthylcellulose.

8. Utilisation selon la revendication 1, dans lequel la solution comprend 0,1 % en poids à 2 % en poids de propylène glycol.
